# EUROPEAN PATENT SPECIFICATION

(11) **EP 4 272 641 B1**
(45) Date of publication and mention of the grant of the patent: **02.12.2026**
(21) Application number: 22795939.2
(22) Date of filing: 25.02.2022
(51) Int. Cl.: A61B 5/16, A61B 5/00, A61B 5/11, A61B 5/0205, G16H 20/70, A61M 21/02

(54) **ELECTRONIC DEVICE FOR DETERMINING PANIC DISORDER, AND OPERATION METHOD THEREOF**
ELEKTRONISCHE VORRICHTUNG ZUR BESTIMMUNG EINER PANIKSTÖRUNG UND BETRIEBSVERFAHREN DAFÜR
DISPOSITIF ÉLECTRONIQUE POUR DÉTERMINER UN TROUBLE PANIQUE, ET SON PROCÉDÉ DE FONCTIONNEMENT

(30) Priority: 30.04.2021 KR 20210056853
(43) Date of publication of application: 08.11.2023
(73) Proprietor: Samsung Electronics Co., Ltd., Suwon-si, Gyeonggi-do 16677 (KR)
(72) Inventor: LEE, Hongji, Suwon-si, Gyeonggi-do 16677 (KR); PARK, Jaehyuck, Suwon-si, Gyeonggi-do 16677 (KR); LEE, Donghyun, Suwon-si, Gyeonggi-do 16677 (KR); HWANG, Minkyung, Suwon-si, Gyeonggi-do 16677 (KR)
(74) Representative: Appleyard Lees IP LLP
(86) International application number: PCT/KR2022/002806
(87) International publication number: WO 2022/231105

(56) References cited:
- WO-A1-2020/254127
- CN-A- 112 297 023
- DE-A1- 102018 200 517
- KR-A- 20170 061 263
- KR-A- 20170 093 470
- KR-A- 20190 043 319
- KR-A- 20200 120 105
- CHOI, DONG-OUN: " Panic Disorder Symptom Care System Based on Context Awareness. ", JOURNAL OF THE KOREA ENTERTAINMENT INDUSTRY ASSOCIATION, vol. 13, no. 4, 1 June 2019 (2019-06-01), pages 63 - 70, XP055982697

## Description

### Technical Field

The present disclosure relates to biometric detection via electronic devices, and, more particularly, to electronic device-facilitated detection of panic attacks.

### Background Art

In recent times, smartphones and smart wearable devices have become widespread. The functions provided by such services has diversified rapidly. For example, there has been proliferation in health-management applications integrated with applications and devices which can monitor biological signals. In recent times, mental health, anxiety and stress from the COVID-19 pandemic has emerged as an important social issue. The World Health Organization (WHO) and World Federation of Mental Health (WFMH) have established October 10th as World Mental Health day, in hopes of promoting mental health and improving public awareness of mental illness.

Representative mental disorders include panic disorder (e.g., panic attacks), depression, bipolar disorder, and schizophrenia. Early detection and treatment of these disorders is often highly beneficial to patients
A "panic attack" is as anxiety disorder in which panic-like symptoms occur in ordinary situations that are not objectively dangerous. Sufferers may suddenly experience extreme fear and anxiety in such an attack. Panic attacks are difficult to predict and may occur. During such a panic attack, after onset, the condition typically worsens, reaching a peak at about 10 minutes from onset, continues then for about 20-30 minutes, and then dissipates. Panic attacks rarely last for longer than an hour. Therefore, even if a panic attack occurs, it may be difficult to diagnose as any physiological abnormality may have disappeared by the time it can be tested for.

Physical symptoms corresponding to a panic attack are as follows, and four or more symptoms occur suddenly.
- Feeling short of breath or choking;
- Feeling dizzy, trembling, or fainting;
- You have a rapid heartbeat or a fast heartbeat;
- Your hands and feet or body tremble;
- Sweating;
- Feeling like someone is choking;
- Feeling nauseous or vomiting;
- I feel like I am in another world or I feel like I am not me;
- Hands and feet tingle or I feel my hands and feet numb;
- Feeling hot or chilled;
- Feeling pain or discomfort in a chest area;
- Feeling the fear of dying; and
- Feeling fear of going crazy or losing control.
- Further, there are three major treatment methods for panic disorder. These are:
- Medication;
- Cognitive behavioral therapy (i.e., cognitive therapy that corrects various prejudices that patients misunderstand or misbelieve, and behavioral therapy to help patients access a feared place or situation without anxiety); and
- Treatment to self-regulate physical symptoms that occur during panic attacks by training breathing control/muscle relaxation.

DE102018200517A1 discloses a method for reporting a panic situation, in which the steps detecting (S2) of at least one physiological parameter based on the at least one physiological parameter, determining (S4) whether there is a panic situation, querying (S6) whether a normal situation exists when the panic situation is detected and reporting (S8) the panic situation if the normal situation is not confirmed.

CN112297023A discloses a device that acquires physiological state data through a built-in sensor and uploads the physiological state data to the intelligent management system; as well as receiving emotional guidance recommendation information, and displaying the emotional state.WO2020254127A1 discloses a Virtual Reality (VR) therapeutic system for providing psychological therapy to a patient.

### Disclosure of Invention

### Technical Problem

If a panic attack can be predicted, a patient can be warned via notification of an potential onset of the same. Thus, it is desirable to provide a service capable of detecting and predicting the onset of panic attacks, through the use of wearable devices, biometric monitoring, etc. The prediction and detection service may help a user control, avoid or mitigate the effects of a panic attack. A mobile device such as a smart phone and/or a wearable device may continuously measure a user's bio-signals (e.g., biometrics) to provide various services associated with the same.

The present invention provides an electronic device and related method as defined in the claims that follow. An electronic device and a method of operating the electronic device according to certain embodiments described in this document may determine whether a user is experiencing or will experience a panic attack, based on the user's state information, such as their biometrics, and information indicative of an environment context of the user.

An electronic device according to certain embodiments disclosed herein may determine a panic attack and a present stage of the panic disorder, based on at least one of a user's bio-signal, motion, location, and/or peripheral environment, and provide guidance according to the detected panic disorder stage.

For example, the electronic device disclosed herein may include a biometric sensor such as a photoplethysmogram (PPG) sensor, an electrodermal activity (EDA) sensor, and/or a body temperature sensor to measure a bio-signal. Further, the electronic device may include an acceleration sensor, a gyroscope, a barometer, and/or a geomagnetic sensor to measure a signal related to a user's motion. Further, the electronic device may include a communication module, and obtain information related to a location and/or peripheral environment thereof based on information received from an external electronic device and/or a server. The electronic device disclosed herein may determine a panic disorder stage based on the information. Further, the electronic device may provide a guide according to the panic disorder stage to a user with various methods such as a screen, a voice, and a vibration.

Technical problems to be achieved in this document are not limited to the technical problems mentioned above, and other technical problems not mentioned will be clearly understood by those of ordinary skill in the art to which the disclosure belongs from the description below.

### Solution to Problem

According to certain embodiments of the disclosure, an electronic device includes a memory, storing a panic-disorder model that is user-specific, panic-disorder information, and a plurality of guidance information for each of a plurality of panic-disorder stages, a sensor module, a communication module, a processor operatively connected to the memory, the sensor module, and the communication module, wherein the processor is configured to: detect a state of the user via the sensor module, detect environmental information via the communication module, enter the detected state and the detected environmental information into the panic-disorder model to determine a panic-disorder stage of the user, retrieve and output one of the plurality of guidance information corresponding to the determined panic-disorder stage, and update the panic-disorder model based on the detected state and the detected environmental information

According to certain embodiments of the disclosure, a method of operating an electronic device includes storing, in a memory, a panic-disorder model that is user-specific, panic-disorder information, and a plurality of guidance information for each of a plurality of panic-disorder stages, detecting a state of a user via a sensor module, detecting environmental information via a communication module, entering, by a processor, the detected state and the detected environmental information into the panic-disorder model to determine a panic-disorder stage of the user, retrieving and outputting, via output circuitry, one of the plurality of guidance information corresponding to the determined panic-disorder stage, updating, by the processor, the panic-disorder model based on the detected state and the detected environmental information.

### Advantageous Effects of Invention

For example, the electronic device may predict a time of onset of a panic attack.

For example, the electronic device output a notification warning a user of onset of a panic attack.

For example, the electronic device may determine whether the user has panic disorder.

For example, the electronic device may provide a user with guidance that may prevent worsening of a panic attack related symptom, after a panic attack has occurred.

For example, a panic disorder determination model may be implemented via execution of machine-learning on user state information and device environmental information, when a panic attack is experienced by the user.

For example, if the user follows guidance provided by the device during or proximate to a panic attack, panic attack symptoms and effects can be reduced.

### Brief Description of Drawings

In connection with the description of the drawings, the same or similar reference numerals may be used for the same or similar components.
FIG. 1 is a block diagram illustrating an electronic device in a network environment according to certain embodiments.
FIG. 2 is a block diagram illustrating an electronic device according to certain embodiments.
FIG. 3 is a flowchart illustrating a method for a processor to determine a user's panic disorder according to certain embodiments.
FIG. 4A is a flowchart illustrating a method for a processor to determine a panic disorder stage according to certain embodiments.
FIG. 4B is a graph related to a method for a processor to determine a panic disorder stage according to time and information according to certain embodiments.
FIG. 4C is a graph comparing user state information obtained by a processor in a general case and user state information when panic disorder occurs according to certain embodiments.
FIGS. 5A, 5B, 5C, 5D, and 5E are diagrams illustrating examples of guide information provided by a processor according to certain embodiments.

### Mode for the Invention

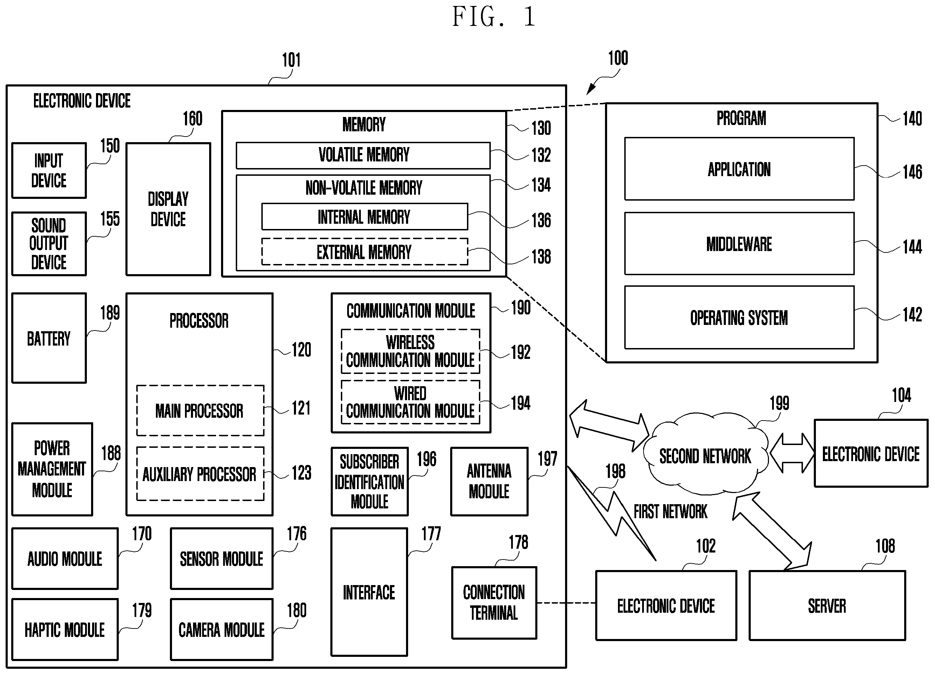

FIG. 1 is a block diagram illustrating an electronic device 101 in a network environment 100 according to certain embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beamforming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element implemented using a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to certain embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or loT-related technology.

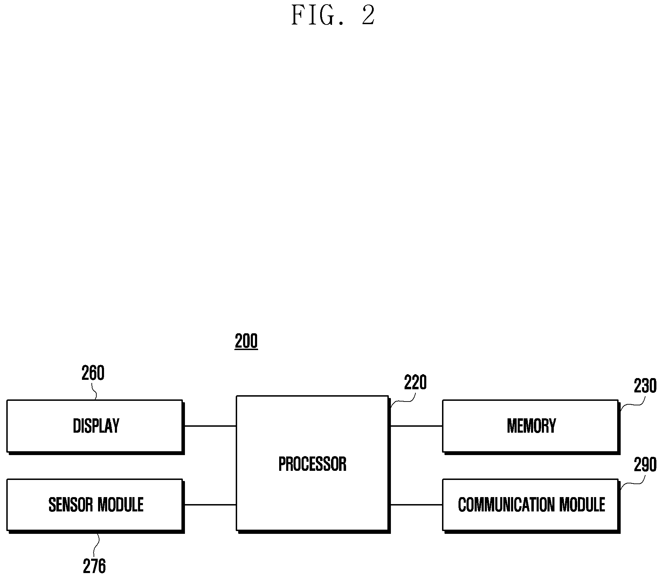

FIG. 2 is a block diagram illustrating an electronic device according to certain embodiments.

Referring to FIG. 2, an electronic device 200 (e.g., the electronic device 101 of FIG. 1) may include a processor 220 (e.g., the processor 120 of FIG. 1), a memory 230 (e.g., the memory 130 of FIG. 1), a display 260 (e.g., the display module 160 of FIG. 1), a sensor module 276 (e.g., the sensor module 176 of FIG. 1), and/or a communication module 290 (e.g., the communication module 190 of FIG. 1). The components included in FIG. 2 may in some embodiments be those included within the electronic device 200, but the electronic device 200 may include various other components, such as those illustrated in FIG. 1.

The sensor module 276 may measure a signal related to a user's state including a user's motion and/or biometric information. According to certain embodiments, the sensor module 276 may include a body temperature sensor and/or a biometric sensor such as a PPG sensor, and/or an EDA sensor (or a motion-related biometric sensor).

According to an embodiment, the motion sensor may measure a signal related to a user's motion. For example, the motion sensor may include an acceleration sensor, a gyroscope, a barometer, and/or a geomagnetic sensor. The processor 220 may obtain the user's motion information such as stationary, sedentary, walking, running, and/or falling motion based on the signal measured by the motion sensor.

According to an embodiment, the photoplethysmogram (PPG) sensor may measure a signal related to a change in the user's blood. For example, by measuring a change in an amount of transmitted light according to a change in blood flow in a peripheral blood vessel by repeating contraction and relaxation of the heart, the PPG sensor may measure a change in blood volume in a blood vessel. For example, the PPG sensor may include a receiver (e.g., photodiode) for converting light energy into electrical energy and an emitter (e.g., LED) for converting electrical energy into light energy. The LED may emit light of wavelengths of infrared (IR) and/or visible light (red, blue, green). The emitter may emit light using electrical energy, and when light emitted from the emitter is transmitted to a skin, the receiver may detect the remaining reflected light after being partially absorbed by the skin. According to an embodiment, when the PPG sensor is brought into contact with a user's body part (e.g., finger) and the contact is maintained for a specified time or longer, the blood volume of the blood vessel of the body part changes by contraction and relaxation of the heart and thus the PPG sensor may measure a change in an amount of light reflected from the finger. For example, during a systole of the heart, blood flows into the blood vessels; thus, a volume of blood in the fingers increases, and as an absorption amount of light emitted from the emitter increases, an amount of reflected light decreases; thus, an amount of light detected by the receiver decreases. For example, during the diastole of the heart, blood in the blood vessels decreases; thus, a volume of blood in the fingers decreases, and as an absorption amount of light emitted from the emitter is reduced, an amount of reflected light increases; thus, an amount of light detected by the receiver increases.

According to an embodiment, the processor 220 may determine a blood pressure, blood sugar, a heart rate, and/or a blood volume based on a signal related to a change in blood volume in a blood vessel measured by the PPG sensor. For example, the processor 220 may count the number of peak-to-peak for a specified time in a signal measured through the PPG sensor or convert the measured signal into a frequency domain to determine a pulse based on a main peak value within a frequency range of a bio-signal. For example, the processor 220 may determine a stress index based on a change in an interval between peak-to-peak in the signal measured through the PPG sensor. For example, the processor 220 may analyze a peak envelope (e.g., empirical mode decomposition (EMD) and/or wavelets method) in a signal measured through the PPG sensor to determine a respiration index. For example, the processor 220 may analyze a feature point from a waveform of a signal measured through the PPG sensor to determine a blood pressure.

According to an embodiment, the electrodermal activity (EDA) sensor may measure a signal related to skin conductivity. For example, the electrodermal activity (EDA) sensor may measure a signal related to skin resistance by sweat gland activity. For example, when sweat gland activity increases, the increased quantity of sweat also increases skin conductance and to decrease skin resistance. According to an embodiment, the EDA sensor may be configured with two electrodes, and each electrode thereof may be attached to a human body. The EDA sensor may apply a very small constant voltage to the two electrodes in contact with the body and measure a flowing current to measure skin conductivity. Skin conductance, galvanic skin response (GSR), and electrodermal activity (EDA) may all mean the same technology. According to an embodiment, the processor 220 may determine an amount of sweat based on a signal related to skin conductivity measured by the EDA.

According to an embodiment, the body temperature sensor may measure thermal radiation emitted from a person body using an infrared thermometer (IRT). According to an embodiment, the processor 220 may determine a body temperature based on thermal radiation energy measured by the body temperature sensor.

The communication module 290 may communicate with an external electronic device (e.g., the electronic device 102 and/or the electronic device 104 of FIG. 1) and/or a server (e.g., the server 108 of FIG. 1) through a network (e.g., the first network 198 and/or the second network 199 of FIG. 1) to receive and/or transmit various types of information. For example, the communication module 290 may communicate with an external electronic device and/or a server using a global navigation satellite system (GNSS) (e.g., global positioning system (GPS) and/or GLONASS), Bluetooth, Bluetooth low energy (BLE), Wi-Fi, ultra-wide band (UWB), and/or near field communication (NFC) technology to receive and/or transmit various types of information. The processor 220 may be connected to the communication module 290 to process various types of information received by the communication module 290 from the external electronic device and/or the server. Further, the processor 220 may control the communication module 290 to transmit various types of information to the external electronic device and/or the server.

The communication module 290 according to certain embodiments may obtain information related to a peripheral environment and/or a location of the electronic device 200.

According to an embodiment, the communication module 290 may receive information related to a location of the electronic device 200. For example, the communication module 290 may receive information related to a location of the electronic device 200 from the external electronic device and/or the server using GNSS, Bluetooth, Bluetooth low energy (BLE), Wi-Fi, ultra-wide band (UWB) and/or near field communication (NFC) technology. For example, the processor 220 may obtain a location of the electronic device 200 based on information received by the communication module 290.

According to an embodiment, the communication module 290 may receive information related to a peripheral environment of the electronic device 200. For example, the communication module 290 may receive identification information of an external electronic device positioned in the vicinity of the electronic device 200. The processor 220 may analyze a peripheral environment based on the identification information of the external electronic device received by the communication module 290. For example, the processor 220 may determine that the user is in an environment with a high population density corresponding to the number of identification information of the external electronic device received by the communication module 290 being equal to or greater than the specified number. For example, the processor 220 may determine that the user is in an environment in which the user's visit history is equal to or greater than the specified number of times corresponding to the number included in the identification information list stored in the memory 230 among identification information of an external electronic device received by the communication module 290 being equal to or greater than the specified number.

The display 260 according to an embodiment may be connected to the processor 220 to visually display information processed by the processor 220.

The memory 230 according to an embodiment may temporarily and/or non-temporarily store at least one of information related to panic disorder including a list of identification information of the external electronic device and information related to a panic disorder occurrence place, a panic disorder determination model for classifying a user's panic disorder state into a plurality of stages, or guide information corresponding to each of a plurality of panic disorder stages.

According to an embodiment, information related to a panic disorder occurrence place may be information related to a geographic location where the user has panic disorder. For example, the processor 220 may obtain a panic disorder occurrence location by the user's input and/or or may obtain as a panic disorder occurrence location, a location of the electronic device 200 obtained from the communication module 290 corresponding to the processor 220 determining that panic disorder has occurred in the user.

According to an embodiment, the identification information list of the external electronic device may be a list of information related to the external electronic device having a history connected to the electronic device 200 through the communication module 290.

According to an embodiment, the panic disorder determination model may be a learning model for determining a panic disorder stage in which a user's panic disorder degree is divided into a plurality of stages. According to an embodiment, the panic disorder determination model may determine a panic disorder stage according to the user's panic disorder occurrence degree, and be implemented differently according to the user. For example, the processor 220 may input the user's state information and/or environment information into the panic disorder determination model to output the panic disorder stage. According to an embodiment, the processor 220 may divide the panic disorder stage into four stages according to the user's situation. For example, a panic disorder stage 1 may correspond to a situation in which the user is likely to be exposed to an environment that can cause panic disorder, a panic disorder stage 2 may correspond to a situation in which the user is expected to generate panic disorder, a panic disorder stage 3 may correspond to a situation in which the user has panic disorder, and a panic disorder stage 4 may correspond to a situation after panic disorder occurs in the user. The above-described four stages are an embodiment and may be divided into a plurality of stages instead of four according to certain embodiments.

According to an embodiment, the guide information may be information related to an output alarm corresponding to each panic disorder stage being determined. For example, the guide information may include first guide information corresponding to a panic disorder stage 1, second guide information corresponding to a panic disorder stage 2, third guide information corresponding to a panic disorder stage 3, and fourth guide information corresponding to a panic disorder stage 4.

The processor 220 may include at least one processor and be physically divided. For example, the processor 220 may include a main processor capable of processing a work (or task) utilizing a high performance and/or a secondary processor capable of processing a work (or task) utilizing relatively low power. According to an embodiment, the sensor module 276 may be connected to the secondary processor to measure a signal for 24 hours. According to another embodiment, the processor 220 may switch a state capable of processing a work (or task) utilizing a high performance based on a condition and a state capable of processing a work (or task) utilizing relatively low power to perform processing.

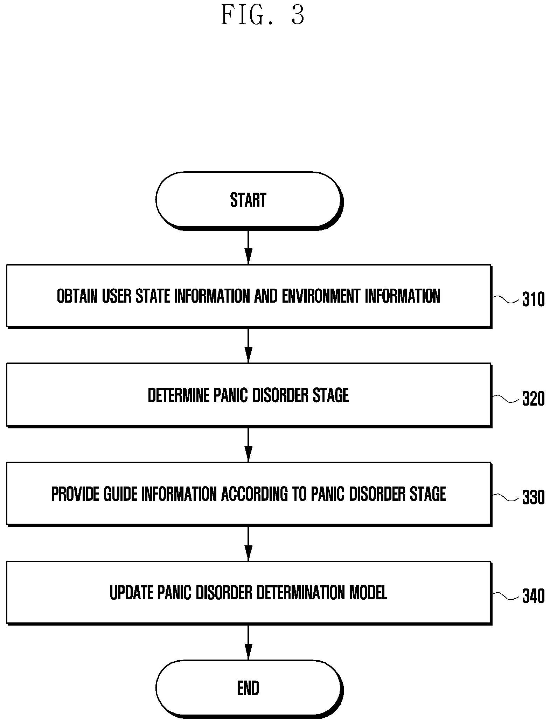

FIG. 3 is a flowchart illustrating a method for a processor (e.g., the processor 220 of FIG. 2) to determine a user's panic disorder according to certain embodiments.

According to certain embodiments, the processor 220 may obtain user state information and environment information in operation 310.

According to an embodiment, the processor 220 may obtain the user's state information including movement of the user (e.g., the user's motion information) and/or the user's biosignal(s) (e.g., the user's biometric information). For example, the processor 220 may obtain the user's state information based on a signal related to the user's biometric information, and/or the user's motion information, as measured by the motion sensor of the sensor module 276.

According to an embodiment, the processor 220 may detect the user's motion information via a signal generated from the sensor module 276 by the user's movements. For example, the sensor module 276 may include an acceleration sensor, a gyroscope, a barometer, and/or a geomagnetic sensor. The processor 220 may obtain the user's motion information such as stationary, sedentary, walking, running, and/or falling motion based on the signal measured by the sensor module 276.

According to an embodiment, the processor 220 may obtain the user's biometric information based on a biometric signal as measured by the sensor module 276. For example, the sensor module 276 may include a PPG sensor, an EDA sensor, and/or a body temperature sensor. The processor 220 may obtain the user's biometric information including at least one of a pulse, a stress, respiration, a blood pressure, a body temperature, and/or an amount of sweat based on the signal measured by the sensor module 276. For example, the processor 220 may determine a pulse, a stress, respiration, and/or a blood pressure based on a signal measured by the PPG sensor. For example, the processor 220 may determine an amount of sweat based on a signal measured by the EDA sensor. For example, the processor 220 may determine the user's body temperature based on a signal measured by the body temperature sensor.

According to an embodiment, the processor 220 may obtain environment information including a location (e.g., location information) and/or peripheral external electronic device information (e.g., information on devices within a certain communicative range of the electronic device 200) from a communication module (e.g., the communication module 290 of FIG. 2). For example, the communication module 290 may communicate with an external electronic device and/or the server using GNSS, Bluetooth, Bluetooth low energy (BLE), Wi-Fi, ultra-wide band (UWB), and/or near field communication (NFC) technology to receive information related to a location of the electronic device 200 and/or information related to a peripheral network environment of the electronic device 200 and other external devices thereof.

According to an embodiment, the communication module 290 may receive information related to a location of the electronic device 200. For example, the location of the electronic device 200 may be received from an external electronic device and/or the server using GNSS, Bluetooth, Bluetooth low energy (BLE), Wi-Fi, ultra-wide band (UWB) and/or near field communication (NFC) technology. For example, the processor 220 may obtain a location of the electronic device 200 based on information received via the communication module 290.

According to an embodiment, the processor 220 may obtain peripheral information based on the information received by the communication module 290. For example, the communication module 290 may obtain identification information of an external electronic device positioned in the vicinity of the electronic device 200. The processor 220 may analyze a peripheral environment based on the identification information of the external electronic device obtained by the communication module 290. For example, the processor 220 may determine that the user is in an environment with a high population density corresponding to the number of identification information of the external electronic device obtained by the communication module 290 being equal to or greater than the specified number. For example, the processor 220 may determine that the user is in an environment in which the user's visit history is equal to or greater than the specified number of times corresponding to the number included in the identification information list stored in the memory 230 among identification information of an external electronic device obtained by the communication module 290 being equal to or greater than the specified number.

According to certain embodiments, the processor 220 may determine a panic disorder stage (e.g., a present stage of panic attack from among a plurality of predefined panic disorder stages) in operation 320.

According to an embodiment, the processor 220 may determine a panic disorder stage based on the user's state information and/or environment information. For example, the processor 220 may input the user's state information and/or environment information into the panic disorder determination model to determine a panic disorder stage.

According to an embodiment, prior to detection, panic disorders may be classified into predetermined stages as organized within a panic disorder determination model. For example, the processor 220 may classify the panic disorder into four stages.

According to an embodiment, a panic disorder stage 1 may correspond to a situation in which a user is likely to be exposed to an environmental factor which may trigger a panic attack For example, the processor 220 may obtain information related to historic panic attacks in the memory 230, such as locations in which panic attacks have previous occurred for the user. Further, the processor 220 may obtain the user's expected movement path and/or a present destination based on information related to the user's schedule (e.g., information obtained from a schedule in the user's scheduler and/or SNS postings). The processor 220 may determine the panic disorder stage as a stage 1 when navigational path to the destination will bring the user near or into a location historically associated with panic attacks for the user.

According to an embodiment, a panic disorder stage 2 may correspond to a situation in which panic disorder is predicted to occur in the user. For example, the processor 220 may utilize a weighted value (e.g., a panic disorder occurrence weight) based on detections of the user's state information and/or environmental information. When the weight is equal to or greater than a predetermined value, the process 220 may that a threshold has been met to invoke stage 2 state, indicating a likelihood of a panic attack.

For example, the processor 220 may adjust the panic disorder occurrence weight in the memory 230, based on one or more of a number of factors, including, for example a present location of the electronic device 200 being within a designated distance from a historical panic attack related location as stored in the memory 230, a identificatory number of an external electronic device detected via the communication module 290, determination that an identificatory number of an external device is included in an identification information list stored in the memory 230, detecting that movements of a user indicate a non-exercise state, the sweat rate and body temperature are out of the specified range, and that the duration of activity or indolence is longer than or equal to the specified time, that movements of the user indicate a non-exercise because pulse, respiration rate, and/or stress index are out of a specified range, and/or that the duration of indolence or activity are longer than or equal to a specified time.

According to an embodiment, a panic disorder stage 3 may correspond to a situation in which panic attack is detected in the user. The processor 220 may adjust the panic disorder occurrence weight based on the user's state information, and may determine a present situation as stage 3 when the weight being equal to or greater than another specified value. For example, the processor 220 may set the panic disorder weight when certain changes in pulse, stress, and/or body temperature are outside of a prespecified range.

According to an embodiment, a panic disorder stage 4 may indicate a post panic attack state. The processor 220 may determine whether the panic attack has subsided, based on the user's state information.

The above-described four stages are an embodiment, and may be divided into a plurality of stages instead of four according to certain embodiments.

For example, the processor 220 may determine whether the panic attack has abated in the user, based on analysis of the user's state information as monitored through stages 1 to 3, and a result of comparing the user's state information as obtained from the sensor module 276.

For example, the processor 220 may determine that the user is still experience a panic attack, when certain biological markers within the user's state information are not reduced by a prespecified range for a specified time or more. According to an embodiment, the processor 220 may obtain the user's motion information via the sensor module 276. For example, from the obtained motion information, the processor 220 may determine whether the user has fallen. For example, subsequently, the processor 220 may execute an operation pre-designated for emergencies, such as a serious fall. The operation may include transmitting location information of the electronic device 200 to an emergency center, transmitting a help request message to the emergency center, calling an ambulance and/or providing an alarm to an external user that is disposed within a specified range from the electronic device 200) and communicatively connected to the same.

For example, the processor 220 may determine that the panic attack has abated when biological markers in the user's state information are reduced beyond prespecified ranges for a specified time or more, as at least partly based on analysis of the user's state information obtained through stages 1 to 3.

According to an embodiment, the processor 220 may adjust a period for which the sensor module 276 detects biometric information based on the user's state information and/or environment information.

For example, the processor 220 may cause the sensor module 276 to detect biometric information within a specified period when the user's movements indicate a non-exercise state, when a sweat amount, pulse, stress level, and/or respiration rate are outside of a prespecified range, and a present duration (e.g., of the state) is longer than or equal to a prespecified time. For example, the processor 220 may control the sensor module 276 to detect biometric information within a specified period in which the electronic device 200 is located within a designated distance from a location historically associated with a panic attack as the memory 230. For example, the processor 220 may control the sensor module 276 to detect biometric information within a specified period when detecting a number of external electronic devices that are not identified with an identification information list in memory, in which a count of the detected number of external electronic devices is equal to or greater than a specified number. For example, the processor 220 may control the sensor module 276 to detect biometric information within a specified period in which the panic disorder stage is determined to be stage 2, stage 3, and/or stage 4. For example, the processor 220 may control the sensor module 276 to detect biometric information within a basic or normalized period in which it is determined that the panic attack has abated and the user is returning to a normal biological state. According to an embodiment, the designated period may be a shorter period than the basic period.

According to certain embodiments, the processor 220 may provide guide information according to the determined panic disorder stage in operation 330.

According to an embodiment, the processor 220 may provide guide information according to each respective stage of panic disorder stage, using a variety of alarms.

For example, the processor 220 may provide the user with first guide information corresponding to stage 1 panic disorder. For example, the processor 220 may provide to output guidance recommending bringing medicine. The guidance may be a visual notification displayed on the display 260 and/or a voice form output through the sound output module (e.g., the sound output module 155 of FIG. 1).

For example, the processor 220 may provide the user with second guide information corresponding to stage 2 panic disorder. For example, the processor 220 may output a breathing control guide, and/or a stretching guide to the user in a voice format through the sound output module (e.g., speaker) and/or in a visual format displayed on the display 260.

For example, the processor 220 may provide third guide information to the user corresponding to a stage 3 panic disorder. For example, the processor 220 may output the breathing control guide and/or the medication recommendation to the user in a voice format through the sound output module, and/or in a visual format on the display 260. For example, the processor 220 may initiate a call to a caregiver and/or provide a some notification thereto indicating that the user is presently experiencing a panic attack.

For example, the processor 220 may provide fourth guide information to the user corresponding to stage 4 panic disorder. For example, the processor 220 may provide a guide . According to an embodiment, the processor 220 may retrieve and/or generate a survey prompting a user to provide feedback on the guidance provided during the panic attack, after detecting that the panic attack has abated. According to an embodiment, the processor 220 may store the user's state information, environment information, and/or survey contents in the memory 230.

According to certain embodiments, the processor 220 may update the panic disorder determination model in operation 340 based on the collected data.

According to an embodiment, the processor 220 may update the panic disorder determination model based on the user's state information and/or environment information. For example, the processor 220 may apply the detected user state information and/or environment information detected in operation 310 to the panic disorder determination model. For example, the processor 220 may update an element related to a panic disorder occurrence weight, based on the detected state information and/or environment information from operation 310. For example, the processor 220 may update an element related to the panic disorder occurrence weight based on the survey content obtained in operation 330.

According to an embodiment, Equation 1 may be utilized to generate a panic disorder occurrence weight "y."

In Equation 1, a may be a weight element, and "t" may be duration. In Equation 1, △pulse may be an amount of change in pulse, △respiration rate may be an amount of change in respiration rate, △stress may be an amount of change in stress index, △sweat amount may be an amount of change in sweat, △body temperature may be an amount of change in body temperature, △environment information may be an amount of change in environment information. According to an embodiment, a criterion for calculating a change amount may be an average value of each index in a previous stage. According to another embodiment, a criterion for calculating the change amount may be an average value of each index in a general case. [Equation 1] 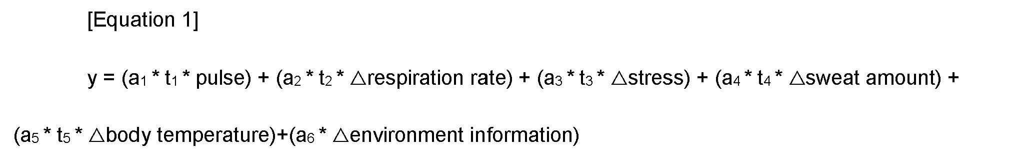 y = (a1 * t1 * pulse) + (a2 * t2 * △respiration rate) + (a3 * t3 * △stress) + (a4 * t4 * △sweat amount) + (a5 * t5 * △body temperature)+(a6 * △environment information)

For example, the processor 220 may update a weight element a in Equation 1 for obtaining a panic disorder occurrence weight y based on at least one of the user's obtained state information, environment information, or feedback to a survey and the effect of a guide.

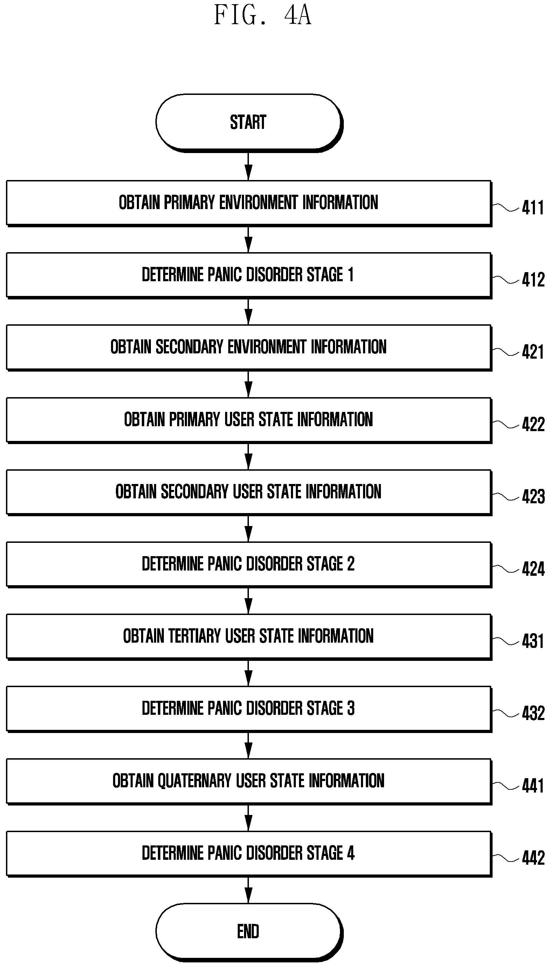

FIG. 4A is a flowchart illustrating a method for a processor (e.g., the processor 220 of FIG. 2) to determine a panic disorder stage according to certain embodiments.

According to certain embodiments, the processor 220 may obtain primary environment information in operation 411.

According to an embodiment, the primary environment information may be information for determining a possibility that the user will be exposed to an environment in which panic disorder may occur. For example, the processor 220 may obtain primary environment information including the user's expected movement path and/or destination information based on information related to the user's schedule (e.g., as determined from the user's social network service (SNS) and/or a calendar items stored in the user's scheduler application).

According to certain embodiments, the processor 220 may determine a panic disorder stage 1 in operation 412.

For example, the panic disorder stage 1 may correspond to a situation in which the user is likely to be exposed to an environment in which panic disorder may occur. The processor 220 may obtain information related to a historic locations in which panic attacks have occurred for the user from information related to panic disorders stored in the memory 230. Further, the processor 220 may determine the panic disorder stage to be a stage 1 when a present location, proximate location or a location encountered during navigation to a destination is included among the stored historic locations.

According to certain embodiments, the processor 220 may obtain secondary environment information in operation 421.

According to an embodiment, the secondary environment information may include location information and/or peripheral information of the electronic device 200 as detected via a communication module (e.g., the communication module 290 of FIG. 2).

According to an embodiment, the communication module 290 may receive information related to a location of the electronic device 200. For example, the communication module 290 may receive information related to a location of the electronic device 200 from an external electronic device and/or the server using GNSS, Bluetooth, Bluetooth low energy (BLE), Wi-Fi, ultra-wide band (UWB), and/or near field communication (NFC) technology. For example, the processor 220 may obtain a location of the electronic device 200 based on information received by the communication module 290.

According to an embodiment, the processor 220 may obtain peripheral information based on the information received by the communication module 290. For example, the communication module 290 may obtain identification information of an external electronic device disposed in the vicinity of the electronic device 200. The processor 220 may analyze a local environment of the electronic device based on the identification information of the external electronic device obtained by the communication module 290. For example, the processor 220 may determine that the user is in an environment with a high population density based on a count of external electronic device identifications detected by the communication module 290 being equal to or greater than the designated number. For example, the processor 220 may determine that the user is in a location in which the user has visited equal to or greater than the specified number of times, corresponding to number of identification information of the external electronic device included in the identification information list is equal to or greater than the specified number.

According to certain embodiments, the processor 220 may obtain primary user state information in operation 422.

According to an embodiment, the processor 220 may obtain primary user state information including the user's motion information and/or the user's biometric information based on the signal measured by the sensor module (e.g., the sensor module 276 of FIG. 2).

For example, the sensor module 276 may include an acceleration sensor, a gyroscope, a barometer, and/or a geomagnetic sensor. The processor 220 may obtain the user's motion information which may indicate to the device whether the user is stationary, sedentary, walking, running, and/or falling, based on the signal measured by the sensor module 276. For example, the sensor module 276 may include a body temperature sensor and/or an EDA sensor. For example, the processor 220 may obtain a body temperature and/or a sweat amount based on a signal obtained from the sensor module 276.

According to an embodiment, the processor 220 may adjust a period in which the sensor module 276 senses biometric information based on the user's state information and/or environment information.

For example, the processor 220 may control the sensor module 276 to sense biometric information within a designated period in which the user's motion indicates a non-exercise state, the sweat amount and/or body temperature change is out of the specified range, and a certain duration (e.g. of the non-exercise state) is longer than or equal to the specified time. For example, the processor 220 may control the sensor module 276 to sense biometric information within a designated period in which a location of the electronic device 200 is disposed within a predetermined distance of a historical location associated with panic attacks, as stored in the memory 230. For example, the processor 220 may control the sensor module 276 to detect biometric information within a specified period when discovering external electronic devices having identification information not included within an identification information list stored in memory, or when a count of the external electronic devices is equal to or greater than a specified count.

According to certain embodiments, the processor 220 may obtain secondary user state information in operation 423.

According to an embodiment, the processor 220 may obtain secondary user state information corresponding to primary user state information that is included in a specified range (e.g., detecting that a sweat amount is higher than a specified value, and/or a body temperature is lower than a specified value).

According to an embodiment, the processor 220 may obtain secondary user state information including a pulse, a respiration rate, and/or a stress index based on a signal measured by the sensor module 276. For example, the sensor module 276 may include a PPG sensor, and the processor 220 may obtain a pulse, a respiration rate, and/or a stress index based on a PPG signal measured by the sensor module 276.

According to certain embodiments, the processor 220 may determine a panic disorder stage 2 in operation 424.

According to an embodiment, the panic disorder stage 2 may correspond to a situation in which panic disorder is predicted to occur in the user.

According to an embodiment, the processor 220 may adjust a panic disorder occurrence weight based on the primary user state information, the secondary user state information and/or environment information, and determine that a present situation corresponds to stage 2 when the weight is equal to or greater than the specified value.

According to an embodiment, the processor 220 may predict onset of a panic attack based on the user's motion, body temperature, and/or sweat amount. For example, the processor 220 may adjust the panic disorder occurrence weight based on a present location of the electronic device 200 when disposed within a threshold distance from the a historic location pre-associated with panic attacks as stored in the memory 230. For example, the processor 220 may adjust the panic disorder occurrence weight based on detecting a threshold number of external electronic devices in the vicinity of the present location. For example, the processor 220 may adjust the panic disorder occurrence weight based on detecting, via user motion, absence of an exercise state, a sweat amount and body temperature being out of a prespecified range, and the duration (e.g., of the present non-exercise state) being longer than or equal to the specified time. For example, the processor 220 may adjust the panic disorder occurrence weight based on detecting a non-exercise state, a pulse rate, respiration rate, and/or stress index being out of a prespecified range, and the duration (e.g., of the present non-exercise state) being longer than or equal to the specified time.

According to certain embodiments, the processor 220 may obtain tertiary user state information in operation 431.

According to an embodiment, the processor 220 may obtain tertiary user state information including the user's motion information and/or the user's biometric information from the sensor module 276.

According to an embodiment, the processor 220 may obtain tertiary user state information including the user's motion, pulse, stress index, and/or body temperature based on the signal measured by the sensor module 276. For example, the sensor module 276 may include a PPG sensor, and the processor 220 may obtain a pulse and/or a stress index based on the PPG signal measured by the sensor module 276. For example, the sensor module 276 may include a body temperature sensor, and the processor 220 may obtain a body temperature from the sensor module 276.

According to certain embodiments, the processor 220 may determine a panic disorder stage 3 in operation 432.

According to an embodiment, the panic disorder stage 3 may correspond to a situation in which the user is experiencing a panic attack.

According to an embodiment, the processor 220 may adjust the panic disorder occurrence weight based on the primary user state information, the secondary user state information, the tertiary user state information, and/or the secondary environment information, and determine a present situation corresponds to stage 3 based on determining that the weight is equal to or greater than the specified value.

For example, the processor 220 may adjust the panic disorder occurrence weight corresponding to the user's motion indicating a non-exercise state, and based on changes in the pulse, stress index, and/or body temperature being outside of a prespecified range.

According to certain embodiments, the processor 220 may obtain quaternary user state information in operation 441.

According to an embodiment, the processor 220 may obtain quaternary user state information related to duration and/or an amount of change in state information of the primary, secondary, and tertiary users.

According to certain embodiments, the processor 220 may determine a panic disorder stage 4 in operation 442.

According to an embodiment, the panic disorder stage 4 may indicate a situation in which the panic attack has abated.

According to an embodiment, the processor 220 may determine whether the panic attack has abated based on changes in the user's state information and/or a duration of the preceding stages. For example, the processor 220 may determine whether the user is in a state out of panic disorder based on the state information of the user.

For example, the processor 220 may determine that the user is still experiencing a panic attack, when the quaternary user state information has not reduced to a prespecified range (e.g., biometric markers sufficiently low as to indicate cessation of the panic attack) and/or for a specified time (e.g., an average length of a panic attack).

According to an embodiment, the processor 220 may obtain the user's motion information based on a signal related to the user's motion obtained from the sensor module 276 corresponding to the user maintaining the panic disorder state. For example, the processor 220 may identify whether the user has fallen according to the user's motion information.

For example, the processor 220 may determine that the user is in a state out of panic disorder corresponding to the state information of the quaternary user being reduced further than the specified range for a specified time or more.

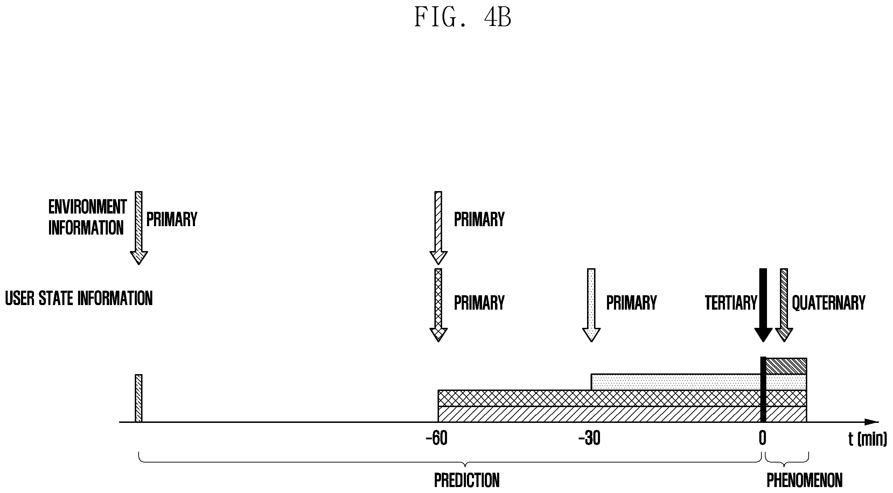

FIG. 4B is a graph related to a method for the processor 220 to determine a panic disorder stage according to a time and information according to certain embodiments.

According to certain embodiments, FIG. 4B is a graph related to a method for the processor 220 to determine a panic disorder stage based on primary environment information, secondary environment information, primary user state information, secondary user state information, tertiary user state information, and/or quaternary user state information according to the flowchart illustrated in FIG. 4A.

According to an embodiment, panic disorder stages 1 and 2 determined based on the primary environment information, the secondary environment information, the primary user state information, and/or the secondary user state information may be a predictive stage of occurrence of panic disorder.

For example, the processor 220 may determine a panic disorder stage 1 corresponding to a situation in which the user is likely to be exposed to an environment in which panic disorder may occur based on the primary environment information.

For example, the processor 220 may determine a panic disorder stage 2 corresponding to a situation in which the user is expected to have panic disorder based on the secondary environment information, the primary user state information, and/or the secondary user state information. Referring to an example graph of FIG. 4B, because it may be identified that panic disorder has occurred in the user about 60 minutes after a time point (e.g., t=-60) at which the processor 220 determines a panic disorder stage 2, the processor 220 may determine the panic disorder stage 2 to predict a time point at which the panic disorder occurs.

For example, the processor 220 may analyze secondary environment information and primary user state information from a time point (e.g., t=-60) at which the panic disorder stage 2 is determined to a time point (e.g., t=-0) at which the panic disorder occurs. Further, the processor 220 may further analyze the secondary user state information from a designated time point (e.g., t=-30) to a time point (e.g., t=0) at which panic disorder occurs.

According to an embodiment, a panic disorder stage 3 determined based on the secondary environment information, the primary user state information, the secondary user state information, and/or the tertiary user state information may be a stage of a time point at which panic disorder occurs.

For example, the processor 220 may determine a panic disorder stage 3 corresponding to a situation in which panic disorder has occurred in the user based on the secondary environment information, the primary user state information, the secondary user state information, and/or the tertiary user state information.

According to an embodiment, a panic disorder stage 4 determined based on the secondary environment information, the primary user state information, the secondary user state information, the tertiary user state information and/or the quaternary user state information may be a stage after occurrence of the panic disorder.

For example, the processor 220 may determine a panic disorder stage 4 corresponding to a situation after panic disorder occurs in the user based on the fourth user state information.

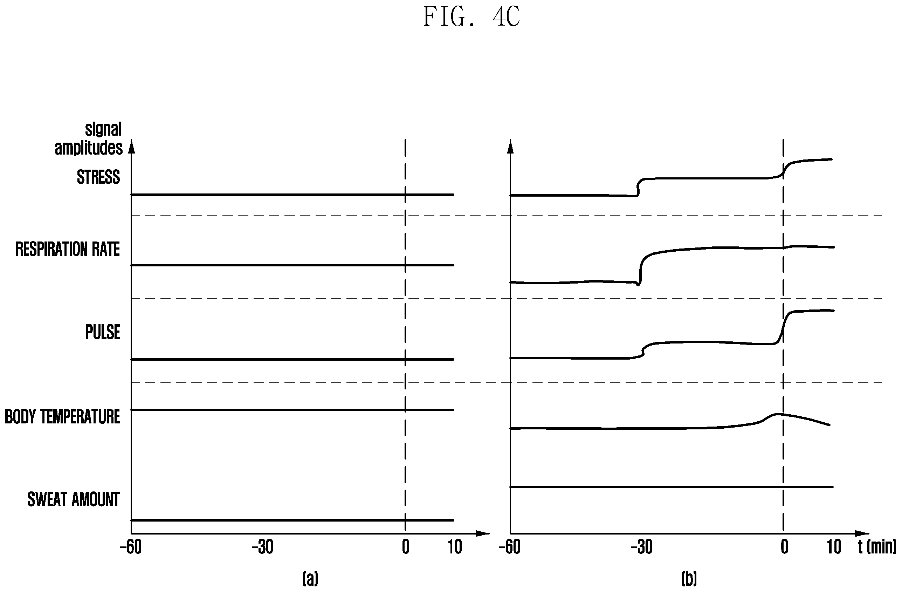

FIG. 4C is a graph comparing user state information obtained by the processor 220 in a general case and user state information in a case in which panic disorder occurs according to certain embodiments.

FIG. 4C(a) is a graph illustrating the user's state (stress, respiration rate, pulse, body temperature, and sweat amount) in a general case, and FIG. 4C(b) is a graph illustrating the user's state when panic disorder occurs.

Referring to the graph (b), it can be seen that a body temperature decreases and a sweat amount increases from about 60 minutes before occurrence of the panic disorder (the time point when t = 0) compared to the graph (a). Further, referring to the graph (b), it can be seen that a stress, a respiration rate, and a pulse index increase from about 30 minutes before occurrence of the panic disorder (the time point when t = 0) compared to the graph (a).

The processor 220 may learn a change in the user's state before panic disorder occurs to predict an occurrence time point of panic disorder.

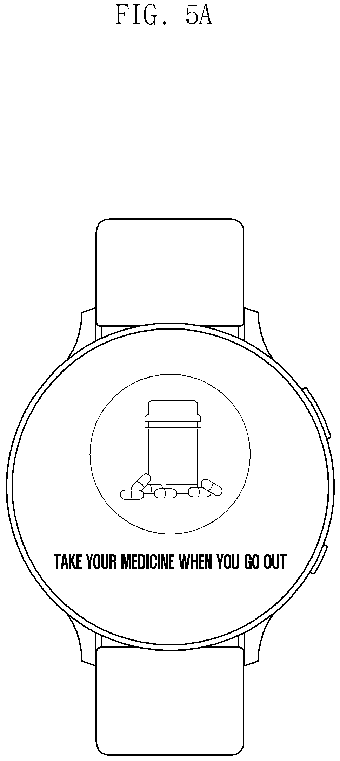

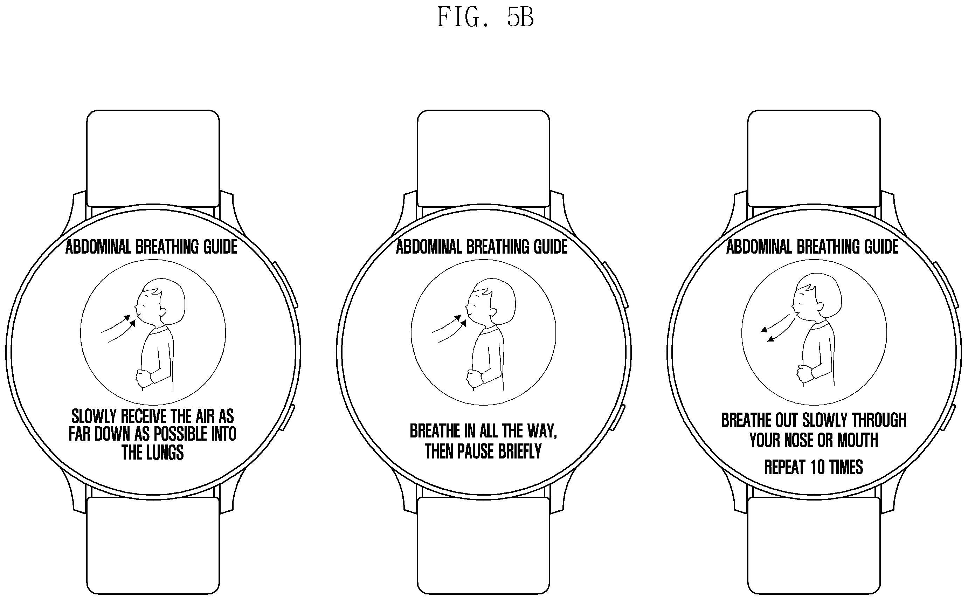

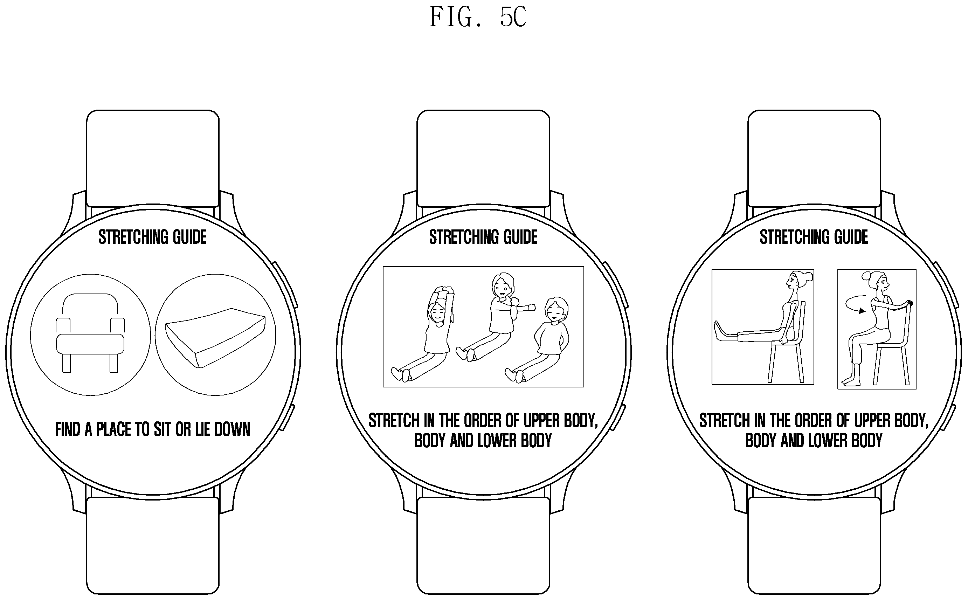

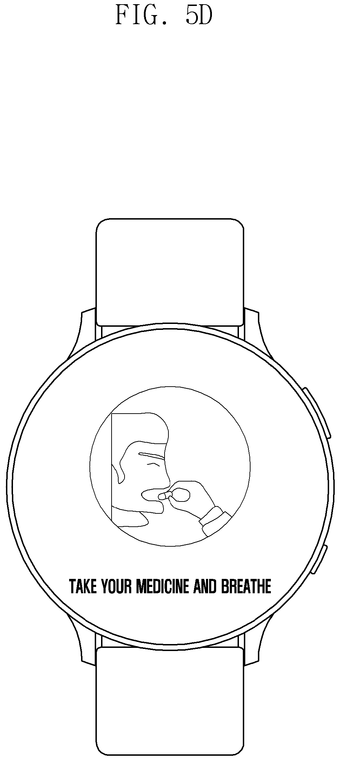

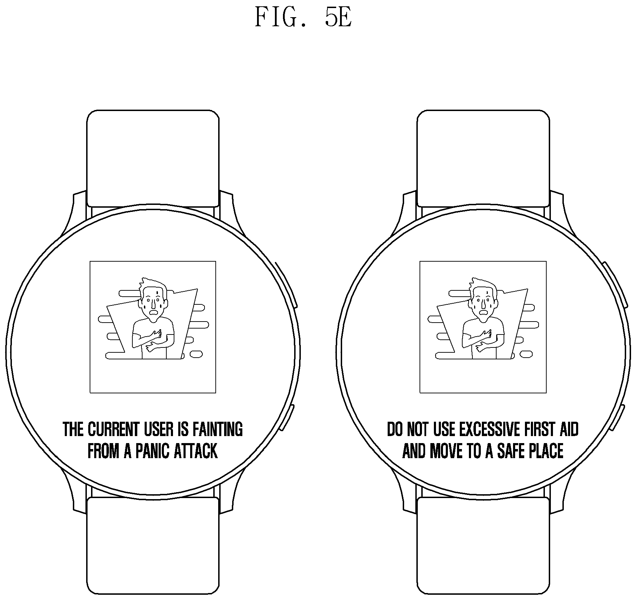

FIGS. 5A, 5B, 5C, 5D, and 5E are diagrams illustrating examples of guide information provided by a processor (e.g., the processor 220 of FIG. 2) according to certain embodiments.

According to certain embodiments, FIG. 5A is a diagram illustrating an example of a first guide provided by the processor 220.

According to an embodiment, the processor 220 may provide a first guide alarm to the user corresponding to the panic disorder stage being a stage 1. For example, the panic disorder stage 1 may correspond to a situation in which the user is likely to be exposed to an environment in which panic disorder are known to occur. For example, the processor 220 may provide a first guide including a guide (e.g., "take your medicine when you go out") to prompt the user to bring medicine before exiting, in a vocal format through the sound output module, and/or in a format of outputting visual information to the display 260.

According to certain embodiments, FIG. 5B is a diagram illustrating an example of a second guide provided by the processor 220.

According to an embodiment, the processor 220 may provide a second guide alarm to the user corresponding to the panic disorder stage being stage 2. According to an embodiment, stage 2 indicates prediction of on oncoming panic attack. For example, the processor 220 may provide a second guide including a breathing control guide (e.g., " Breathe in through your nose. Slowly receive the air as far down as possible into the lungs.", "Breathe in all the way, then pause briefly.", "Breathe out slowly through your nose or mouth. Repeat 10 times.") in the format of visual information displayed to the display 260, and/or in a voice form through the sound output module.

According to certain embodiments, FIG. 5C is a diagram illustrating an example of the second guide provided by the processor 220.

For example, the processor 220 may provide a second guide alarm to the user corresponding to the panic disorder stage being a stage 2. For example, the processor 220 may provide a second guide including a stretching guide (e.g., "find a place to sit or lie down", "stretch an upper body, body, and lower body in order") to the user in a voice form through the sound output module and/or in a form of outputting visual information to the display 260.

According to certain embodiments, FIG. 5D is a diagram illustrating an example of a third guide provided by the processor 220.

For example, the processor 220 may provide a third guide alarm to the user corresponding to the panic disorder stage being a stage 3. For example, the processor 220 may provide a breathing control guide and/or a medication recommendation guide (e.g., "Take your medication and breathe.") to the user in a form of outputting visual information to the display 260 and/or in a voice form through the sound output module.

According to certain embodiments, FIG. 5E is a diagram illustrating an example of a fourth guide provided by the processor 220.

For example, the processor 220 may provide a fourth guide alarm to the user corresponding to the panic disorder stage being stage 4. For example, the processor 220 may provide a guide according to whether the user continues to suffer a panic attack.

For example, the processor 220 may call a designated contact (e.g., caregiver) and/or transmit, output or otherwise provide a guidance notification indicating that the user is suffering a panic attack upon detection of the same.

For example, the processor 220 may execute a function pre-designated for execution in an emergency situation (e.g., send location information of the electronic device 200 to an emergency center, send a help request message to the emergency center, call an ambulance, and/or provide an alarm to an external user connected within a designated range from a location of the electronic device 200), when the emergency situation is detected, such as detecting a panic attack, and/or detecting that the user has fallen, and the user doing not perform a designated action (e.g., touch a screen to cancel an alarm) within a designated time. For example, when the emergency center requests the user's medical information, the processor 220 may decrypt and provide the user's encrypted medical information.

According to an embodiment, the processor 220 may output a feedback survey to be completed by the user upon detecting cessation of the panic attack, to rate the guidance provided by the device. According to an embodiment, the processor 220 may store the user's state information, environment information, and/or survey contents in the memory 230.

The electronic device 200 according to certain embodiments of the disclosure may include a memory 230 configured to store a panic disorder determination model for determining a panic disorder stage according to a user's panic disorder occurrence degree and implemented differently according to the user, information related to panic disorder, and guide information corresponding to each of a plurality of panic disorder stages, a sensor module 276, a communication module 290, and a processor 220 operatively connected to the memory 230, the sensor module 276 and the communication module 290, such that the processor 220 may obtain the user's state information from the sensor module 276, obtain environment information from the communication module 290, input the user's state information and the environment information into the panic disorder determination model to determine a panic disorder stage, provide guide information corresponding to the panic disorder stage, and update the panic disorder determination model based on the user's state information and the environment information.

In the electronic device 200 according to certain embodiments of the disclosure, the sensor module 276 may measure a signal related to a user's motion or user's biometric information, and the biometric information may include at least one of a pulse, a stress, respiration, a blood pressure, and a body temperature, or a sweat amount.

In the electronic device 200 according to certain embodiments of the disclosure, the information related to panic disorder may include information related to a panic disorder occurrence place, the communication module 290 may obtain the environment information including location information of the electronic device 200, and the processor 220 may determine the panic disorder stage based on whether a location of the electronic device 200 is included in the panic disorder occurrence place.

In the electronic device 200 according to certain embodiments of the disclosure, the communication module 290 may scan the periphery to obtain the environment information including identification information of the external electronic device 200, and the processor 220 may determine the panic disorder stage based on the identification information of the external electronic device 200.

In the electronic device 200 according to certain embodiments of the disclosure, the information related to panic disorder may include an identification information list, and the processor 220 may determine the panic disorder stage based on the number of identification information of the external electronic device 200 included in the identification information list.

In the electronic device 200 according to certain embodiments of the disclosure, the processor 220 may control the sensor to adjust a period for sensing the user's state information based on the user's state information or the environment information.

In the electronic device 200 according to certain embodiments of the disclosure, the information related to panic disorder may include information related to a panic disorder occurrence place, and the processor 220 may obtain destination information and provide first guide information of the plurality of guide information corresponding to the destination information being included in the panic disorder occurrence place.

In the electronic device 200 according to certain embodiments of the disclosure, corresponding to the panic disorder stage being a stage corresponding to a user state after occurrence of panic disorder, the processor 220 may perform an action designated to perform in an emergency situation corresponding to obtaining the user's motion information from the sensor module 276 and identifying the user's fall.

In the electronic device 200 according to certain embodiments of the disclosure, the processor 220 may adjust a panic disorder occurrence weight based on the user's state information and/or the environment information, and determine the panic disorder stage based on a range of the panic disorder occurrence weight.

In the electronic device 200 according to certain embodiments of the disclosure, the processor 220 may update an element related to the panic disorder weight based on the user's state information and/or the environment information to update the panic disorder determination model.

A method of operating the electronic device 200 according to certain embodiments of the disclosure may include obtaining user state information from the sensor module 276, obtaining environment information from the communication module 290, determining a panic disorder stage by inputting the user's state information and the environment information into a panic disorder determination model for determining a panic disorder stage according to a user's panic disorder occurrence degree and implemented differently according to the user, providing guide information corresponding to the panic disorder stage, and updating the panic disorder determination model based on the user's state information and the environment information.

The method of operating the electronic device 200 according to certain embodiments of the disclosure may include measuring a signal related to a user's motion or user's biometric information, such that the biometric information may include at least one of a pulse, a stress, respiration, a blood pressure, a body temperature, or a sweat amount.

In the method of operating the electronic device 200 according to certain embodiments of the disclosure, the information related to panic disorder may include information related to a panic disorder occurrence place, and the method may include obtaining the environment information including location information of the electronic device 200 and determining the panic disorder stage based on whether a location of the electronic device 200 is included in the panic disorder occurrence place.

The method of operating the electronic device 200 according to certain embodiments of the disclosure may include obtaining the environment information including identification information of an external electronic device 200 by scanning the periphery and determining the panic disorder stage based on the identification information of the external electronic device 200.

In the method of operating the electronic device 200 according to certain embodiments of the disclosure, the information related to panic disorder may include an identification information list, and the method may include determining the panic disorder stage based on the number of identification information of the external electronic device 200 included in the identification information list.

The method of operating the electronic device 200 according to certain embodiments of the disclosure may include controlling the sensor module 276 to adjust a period for sensing the user's state information based on the user's state information or the environment information.

In the method of operating the electronic device 200 according to certain embodiments of the disclosure, the information related to panic disorder may include information related to a panic attack occurrence place, and the method may include obtaining destination information and providing first guide information of the plurality of guide information corresponding to the destination information being included in the panic attack occurrence place.

The method of operating the electronic device 200 according to certain embodiments of the disclosure may include obtaining the user's action information from the sensor module 276 corresponding to the panic disorder stage being a stage corresponding to a user state after occurrence of panic disorder and performing an action designated to perform in an emergency situation corresponding to identifying the user's fall.

The method of operating the electronic device 200 according to certain embodiments of the disclosure may include adjusting a panic disorder occurrence weight based on the user's state information and/or the environment information and determining the panic disorder stage based on a range of the panic disorder occurrence weight.

The method of operating the electronic device 200 according to certain embodiments of the disclosure may include updating the panic disorder determination model by updating an element related to the panic disorder weight based on the user's state information and/or the environment information.

The electronic device according to certain embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that certain embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with certain embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Certain embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to certain embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to certain embodiments, each component (e.g., a module or a program) of the above-described components may include a single entity or multiple entities, and some of the multiple entities may be separately disposed in different components. According to certain embodiments, one or more of the above-described components may be omitted, or one or more other components may be added. Alternatively or additionally, a plurality of components (e.g., modules or programs) may be integrated into a single component. In such a case, according to certain embodiments, the integrated component may still perform one or more functions of each of the plurality of components in the same or similar manner as they are performed by a corresponding one of the plurality of components before the integration. According to certain embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101), comprising:
a memory (130), storing a panic-disorder model that is user-specific, panic-disorder information, and a plurality of guidance information for each of a plurality of panic-disorder stages;
a sensor module (176);
a communication module (190); and
a processor (120) operatively connected to the memory, the sensor module, and the communication module, wherein the processor is configured to:
detect a state of the user via the sensor module,
detect environmental information via the communication module,
enter the detected state and the detected environmental information into the panic-disorder model to determine a panic-disorder stage of a plurality of panic-disorder stages of the user, wherein the plurality of panic-disorder stages includes a first panic-disorder stage corresponding to a situation in which the user is exposed to an environment that can cause the panic-disorder, a second panic-disorder stage corresponding to a situation in which the user is expected to generate the panic-disorder, a third panic-disorder stage corresponding to a situation in which the user has the panic-disorder, and a fourth panic-disorder stage corresponding to a situation after the panic disorder occurs in the user,
retrieve and output one of the plurality of guidance information corresponding to the determined panic-disorder stage, and
update the panic-disorder model based on the detected state and the detected environmental information.

2. The electronic device of claim 1, wherein the sensor module (176) is configured to measure a signal related to the user's motion or user's biometric information, and
wherein the biometric information includes at least one of a pulse, a stress indicator, a respiration rate, blood pressure, a body temperature, and a sweat rate.

3. The electronic device of claim 1, wherein the panic-disorder information includes historical locations in which panic-attacks previously occurred,
wherein the detected environmental information includes a present location of the electronic device, and
wherein the processor (120) determines panic-disorder stage based on whether the present location is within a designated distance from the historical location panic-disorder information.

4. The electronic device of claim 1, wherein the processor (120) is further configured to: detect the environmental information by scanning, via the communication module, an immediate environment of the electronic device, wherein the environmental information includes identification information of an external electronic device, and
determine the panic disorder stage based on the detected identification information of the external electronic device.

5. The electronic device of claim 4, wherein the panic-disorder information includes a list of device identifications, and
wherein the processor (120) is configured to determine the panic-disorder stage further based on determining whether the detected identification of the external electronic device is included in the list of device identifications.

6. The electronic device of claim 1, wherein the processor is further configured to: adjust, with respect to the sensor (176), a time period for detecting the state of the user, based on the detected state or the detected environmental information.

7. The electronic device of claim 1, wherein the panic-disorder information includes historical locations in which panic-attacks previously occurred, and
wherein the processor (120) is configured to:
obtain a destination location, and
when the destination location corresponds to a particular location included in the panic-disorder information, output one of the plurality of guidance information.

8. The electronic device of claim 1, wherein the processor is configured to:
store, in the memory (130), a function predesignated for automatic execution upon detection of a pre-stored emergency condition;
after detecting onset of a panic-attack based on the detected state of the user, determine whether the determined panic-disorder stage satisfies the pre-stored emergency condition; and
when the determined panic-disorder stage satisfies the pre-stored emergency condition, execute the stored function.

9. The electronic device of claim 1, wherein the processor (120) is configured to:
adjust a panic-disorder weight, based on at least one of the detected state of the user and the detected environmental information,
determine the panic-disorder stage further according to the panic-disorder weight.

10. The electronic device of claim 9, wherein the processor (120) is configured to:
update the panic-disorder model, including changing an element related to the panic-disorder weight based on the detected state and/or the environmental information.

11. A method of operating an electronic device (200), the method comprising:
storing, in a memory (230), a panic-disorder model that is user-specific, panic-disorder information, and a plurality of guidance information for each of a plurality of panic-disorder stages;
detecting a state of a user via a sensor module (276);
detecting environmental information via a communication module (290);
entering, by a processor (220), the detected state and the detected environmental information into the panic-disorder model to determine a panic-disorder stage of a plurality of panic-disorder stages of the user, wherein the plurality of panic-disorder stages includes a first panic-disorder stage corresponding to a situation in which the user is exposed to an environment that can cause the panic-disorder, a second panic-disorder stage corresponding to a situation in which the user is expected to generate the panic-disorder, a third panic-disorder stage corresponding to a situation in which the user has the panic-disorder, and a fourth panic-disorder stage corresponding to a situation after the panic disorder occurs in the user,
retrieving and outputting, via output circuitry, one of the plurality of guidance information corresponding to the determined panic-disorder stage, and
updating, by the processor, the panic-disorder model based on the detected state and the detected environmental information.

12. The method of claim 11, wherein the sensor module (276) is configured to measure a signal related to the user's motion or user's biometric information, and
wherein the biometric information includes at least one of a pulse, a stress indicator, a respiration rate, blood pressure, a body temperature, and a sweat rate.

13. The method of claim 11, wherein the panic-disorder information includes historical locations in which panic-attacks previously occurred,
wherein the detected environmental information includes a present location of the electronic device (200), and
the method further comprise:
determining panic-disorder stage via the panic-disorder model by detecting that the present location matches a historical location included within the panic-disorder information.

14. The method of claim 11, further comprising:
detecting the environmental information by scanning, via the communication module (290), an immediate environment of the electronic device, wherein the environmental information includes identification of an external electronic device, and
determining panic-disorder stage based on the detected identification of the external electronic device.

15. The method of claim 14, wherein the panic-disorder information includes a list of device identifications, and
the method further comprising:
determining panic-disorder stage further based on determining whether the detected identification of the external electronic device is included in the list of device identifications.

## Patentansprüche

1. Elektronische Vorrichtung (101), umfassend:
einen Speicher (130), der ein Panikstörungsmodell, das benutzerspezifisch ist, Panikstörungsinformationen und eine Vielzahl von Führungsinformationen für jede einer Vielzahl von Panikstörungsstufen speichert;
ein Sensormodul (176);
ein Kommunikationsmodul (190); und
einen Prozessor (120), der mit dem Speicher, dem Sensormodul und dem Kommunikationsmodul wirkverbunden ist, wobei der Prozessor zu Folgendem konfiguriert ist:
Erfassen eines Zustands des Benutzers über das Sensormodul,
Erfassen von Umgebungsinformationen über das Kommunikationsmodul,
Eingeben des erfassten Zustands und der erfassten Umgebungsinformationen in das Panikstörungsmodell, um eine Panikstörungsstufe einer Vielzahl von Panikstörungsstufen des Benutzers zu ermitteln, wobei die Vielzahl von Panikstörungsstufen eine erste Panikstörungsstufe, die einer Situation entspricht, in der der Benutzer einer Umgebung ausgesetzt ist, die die Panikstörung verursachen kann, eine zweite Panikstörungsstufe, die einer Situation entspricht, in der erwartet wird, dass der Benutzer die Panikstörung erzeugt, eine dritte Panikstörungsstufe, die einer Situation entspricht, in der der Benutzer die Panikstörung aufweist, und eine vierte Panikstörungsstufe, die einer Situation entspricht, nachdem die Panikstörung beim Benutzer auftritt, beinhaltet,
Abrufen und Ausgeben einer der Vielzahl von Führungsinformationen, die der ermittelten Panikstörungsstufe entspricht, und
Aktualisieren des Panikstörungsmodells basierend auf dem erfassten Zustand und den erfassten Umgebungsinformationen.

2. Elektronische Vorrichtung nach Anspruch 1, wobei das Sensormodul (176) dazu konfiguriert ist, ein Signal zu messen, das sich auf die Bewegung des Benutzers oder die biometrischen Informationen des Benutzers bezieht, und
wobei die biometrischen Informationen mindestens eines von einem Puls, einem Stressindikator, einer Atemfrequenz, Blutdruck, einer Körpertemperatur und einer Schweißfrequenz beinhalten.

3. Elektronische Vorrichtung nach Anspruch 1, wobei die Panikstörungsinformationen historische Standorte beinhalten, an denen Panikattacken zuvor aufgetreten sind,
wobei die erfassten Umgebungsinformationen einen gegenwärtigen Standort der elektronischen Vorrichtung beinhalten, und
wobei der Prozessor (120) die Panikstörungsstufe basierend darauf ermittelt, ob sich der gegenwärtige Standort innerhalb einer bestimmten Entfernung von den Panikstörungsinformationen des historischen Standorts befindet.

4. Elektronische Vorrichtung nach Anspruch 1, wobei der Prozessor (120) ferner zu Folgendem konfiguriert ist: Erfassen der Umgebungsinformationen durch Abtasten einer unmittelbaren Umgebung der elektronischen Vorrichtung über das Kommunikationsmodul, wobei die Umgebungsinformationen Identifizierungsinformationen einer externen elektronischen Vorrichtung beinhalten, und
Ermitteln der Panikstörungsstufe basierend auf den erfassten Identifizierungsinformationen der externen elektronischen Vorrichtung.

5. Elektronische Vorrichtung nach Anspruch 4, wobei die Panikstörungsinformationen eine Liste von Vorrichtungsidentifizierungen beinhalten, und
wobei der Prozessor (120) dazu konfiguriert ist, die Panikstörungsstufe ferner basierend auf dem Ermitteln, ob die erfasste Identifizierung der externen elektronischen Vorrichtung in der Liste der Vorrichtungsidentifizierungen beinhaltet ist, zu ermitteln.

6. Elektronische Vorrichtung nach Anspruch 1, wobei der Prozessor ferner zu Folgendem konfiguriert ist: Einstellen, in Bezug auf den Sensor (176), eines Zeitraums zum Erfassen des Zustands des Benutzers, basierend auf dem erfassten Zustand oder den erfassten Umgebungsinformationen.

7. Elektronische Vorrichtung nach Anspruch 1, wobei die Panikstörungsinformationen historische Standorte beinhalten, an denen Panikattacken zuvor aufgetreten sind, und
wobei der Prozessor (120) zu Folgendem konfiguriert ist:
Erhalten eines Zielstandorts, und
wenn der Zielstandort einem speziellen Standort entspricht, der in den Panikstörungsinformationen beinhaltet ist, Ausgeben einer der Vielzahl von Führungsinformationen.

8. Elektronische Vorrichtung nach Anspruch 1, wobei der Prozessor zu Folgendem konfiguriert ist:
Speichern, in dem Speicher (130), einer Funktion, die für die automatische Ausführung bei Erfassen eines vorab gespeicherten Notfallzustands vorbestimmt ist;
nach dem Erfassen des Einsetzens einer Panikattacke basierend auf dem erfassten Zustand des Benutzers, Ermitteln, ob die ermittelte Panikstörungsstufe die vorab gespeicherte Notfallbedingung erfüllt; und
wenn die ermittelte Panikstörungsstufe die vorab gespeicherte Notfallbedingung erfüllt, Ausführen der gespeicherten Funktion.

9. Elektronische Vorrichtung nach Anspruch 1, wobei der Prozessor (120) zu Folgendem konfiguriert ist:
Einstellen einer Panikstörungsgewichtung basierend auf mindestens einem von dem erfassten Zustand des Benutzers und den erfassten Umgebungsinformationen,
Ermitteln der Panikstörungsstufe ferner gemäß der Panikstörungsgewichtung.

10. Elektronische Vorrichtung nach Anspruch 9, wobei der Prozessor (120) zu Folgendem konfiguriert ist:
Aktualisieren des Panikstörungsmodells, einschließlich Ändern eines Elements, das sich auf die Panikstörungsgewichtung bezieht, basierend auf dem erfassten Zustand und/oder den Umgebungsinformationen.

11. Verfahren zum Betreiben einer elektronischen Vorrichtung (200), wobei das Verfahren Folgendes umfasst:
Speichern, in einem Speicher (230), eines Panikstörungsmodells, das benutzerspezifisch ist, von Panikstörungsinformationen und einer Vielzahl von Führungsinformationen für jede einer Vielzahl von Panikstörungsstufen;
Erfassen eines Zustands eines Benutzers über ein Sensormodul (276);
Erfassen von Umgebungsinformationen über ein Kommunikationsmodul (290);
Eingeben, durch einen Prozessor (220), des erfassten Zustands und der erfassten Umgebungsinformationen in das Panikstörungsmodell, um eine Panikstörungsstufe einer Vielzahl von Panikstörungsstufen des Benutzers zu ermitteln, wobei die Vielzahl von Panikstörungsstufen eine erste Panikstörungsstufe, die einer Situation entspricht, in der der Benutzer einer Umgebung ausgesetzt ist, die die Panikstörung verursachen kann, eine zweite Panikstörungsstufe, die einer Situation entspricht, in der erwartet wird, dass der Benutzer die Panikstörung erzeugt, eine dritte Panikstörungsstufe, die einer Situation entspricht, in der der Benutzer die Panikstörung aufweist, und eine vierte Panikstörungsstufe, die einer Situation entspricht, nachdem die Panikstörung beim Benutzer auftritt, beinhaltet,
Abrufen und Ausgeben, über eine Ausgabeschaltung, einer der Vielzahl von Führungsinformationen, die der ermittelten Panikstörungsstufe entspricht, und
Aktualisieren, durch den Prozessor, des Panikstörungsmodells basierend auf dem erfassten Zustand und den erfassten Umgebungsinformationen.

12. Verfahren nach Anspruch 11, wobei das Sensormodul (276) dazu konfiguriert ist, ein Signal zu messen, das sich auf die Bewegung des Benutzers oder die biometrischen Informationen des Benutzers bezieht, und
wobei die biometrischen Informationen mindestens eines von einem Puls, einem Stressindikator, einer Atemfrequenz, Blutdruck, einer Körpertemperatur und einer Schweißfrequenz beinhalten.

13. Verfahren nach Anspruch 11, wobei die Panikstörungsinformationen historische Standorte beinhalten, an denen Panikattacken zuvor aufgetreten sind,
wobei die erfassten Umgebungsinformationen einen gegenwärtigen Standort der elektronischen Vorrichtung (200) beinhalten, und
wobei das Verfahren ferner Folgendes umfasst:
Ermitteln der Panikstörungsstufe über das Panikstörungsmodell durch Erfassen, dass der gegenwärtige Standort mit einem historischen Standort übereinstimmt, der in den Panikstörungsinformationen beinhaltet ist.

14. Verfahren nach Anspruch 11, ferner umfassend:
Erfassen der Umgebungsinformationen durch Abtasten einer unmittelbaren Umgebung der elektronischen Vorrichtung über das Kommunikationsmodul (290), wobei die Umgebungsinformationen die Identifizierung einer externen elektronischen Vorrichtung beinhalten, und
Ermitteln der Panikstörungsstufe basierend auf der erfassten Identifizierung der externen elektronischen Vorrichtung.

15. Verfahren nach Anspruch 14, wobei die Panikstörungsinformationen eine Liste von Vorrichtungsidentifizierungen beinhalten, und
wobei das Verfahren ferner Folgendes umfasst:
Ermitteln der Panikstörungsstufe ferner basierend auf dem Ermitteln, ob die erfasste Identifizierung der externen elektronischen Vorrichtung in der Liste der Vorrichtungsidentifizierungen beinhaltet ist.

## Revendications

1. Dispositif électronique (101), comprenant :
une mémoire (130), stockant un modèle de trouble panique qui est spécifique à l'utilisateur, des informations de trouble panique, et une pluralité d'informations de guidage pour chacune d'une pluralité de stades de trouble panique ;
un module de capteur (176) ;
un module de communication (190) ; et
un processeur (120) fonctionnellement connecté à la mémoire, au module de capteur et au module de communication, ledit processeur étant configuré pour :
détecter un état de l'utilisateur par l'intermédiaire du module de capteur, détecter des informations environnementales par l'intermédiaire du module de communication, entrer l'état détecté et les informations environnementales détectées dans le modèle de trouble panique pour déterminer un stade de trouble panique d'une pluralité de stades de trouble panique de l'utilisateur, ladite pluralité de stades de trouble panique comprenant un premier stade de trouble panique correspondant à une situation dans laquelle l'utilisateur est exposé à un environnement qui peut causer le trouble panique, un deuxième stade de trouble panique correspondant à une situation dans laquelle l'utilisateur est censé générer le trouble panique, un troisième stade de trouble panique correspondant à une situation dans laquelle l'utilisateur présente le trouble panique, et un quatrième stade de trouble panique correspondant à une situation après que le trouble panique est survenu chez l'utilisateur, récupérer et émettre en sortie l'une de la pluralité d'informations de guidage correspondant au stade de trouble panique déterminé, et
mettre à jour le modèle de trouble panique sur la base de l'état détecté et des informations environnementales détectées.

2. Dispositif électronique de la revendication 1, ledit module de capteur (176) étant configuré pour mesurer un signal lié au mouvement de l'utilisateur ou aux informations biométriques de l'utilisateur, et
lesdites informations biométriques comprenant au moins l'un d'un pouls, d'un indicateur de stress, d'un taux de respiration, d'une pression artérielle, d'une température corporelle et d'un taux de sueur.

3. Dispositif électronique de la revendication 1, lesdites informations de trouble panique comprenant des emplacements historiques dans lesquels des attaques de panique sont survenues précédemment,
lesdites informations environnementales détectées comprenant un emplacement actuel du dispositif électronique, et
ledit processeur (120) déterminant le stade de trouble panique sur la base que l'emplacement actuel se trouve ou non dans les limites d'une distance désignée à partir des informations de trouble panique d'emplacement historique.

4. Dispositif électronique de la revendication 1, ledit processeur (120) étant en outre configuré pour : détecter les informations environnementales en balayant, par l'intermédiaire du module de communication, un environnement immédiat du dispositif électronique, lesdites informations environnementales comprenant des informations d'identification d'un dispositif électronique externe, et
déterminer le stade de trouble panique sur la base des informations d'identification détectées du dispositif électronique externe.

5. Dispositif électronique de la revendication 4, lesdites informations de trouble panique comprenant une liste d'identifications de dispositif, et
ledit processeur (120) étant configuré pour déterminer le stade de trouble panique sur la base en outre de la détermination pour savoir si l'identification détectée du dispositif électronique externe est comprise dans la liste des identifications de dispositif.

6. Dispositif électronique de la revendication 1, ledit processeur étant en outre configuré pour : régler, par rapport au capteur (176), une période de temps pour détecter l'état de l'utilisateur, sur la base de l'état détecté ou des informations environnementales détectées.

7. Dispositif électronique de la revendication 1, lesdites informations de trouble panique comprenant des emplacements historiques dans lesquels des attaques de panique sont survenues précédemment, et
ledit processeur (120) étant configuré pour :
obtenir un emplacement de destination, et
lorsque l'emplacement de destination correspond à un emplacement particulier compris dans les informations de trouble panique, émettre en sortie l'une de la pluralité d'informations de guidage.

8. Dispositif électronique de la revendication 1, ledit processeur étant configuré pour :
stocker, dans la mémoire (130), une fonction prédésignée pour une exécution automatique lors de la détection d'une condition d'urgence préstockée ;
après la détection du début d'une attaque de panique sur la base de l'état détecté de l'utilisateur,
déterminer si le stade de trouble panique déterminé satisfait à la condition d'urgence préstockée ; et
lorsque le stade de trouble panique déterminé satisfait à la condition d'urgence préstockée, exécuter la fonction stockée.

9. Dispositif électronique de la revendication 1, ledit processeur (120) étant configuré pour : régler un poids de trouble panique, sur la base d'au moins l'un de l'état détecté de l'utilisateur et des informations environnementales détectées, déterminer davantage le stade de trouble panique selon le poids de trouble panique.

10. Dispositif électronique de la revendication 9, ledit processeur (120) étant configuré pour : mettre à jour le modèle de trouble panique, comprenant la modification d'un élément lié au poids de trouble panique sur la base de l'état détecté et/ou des informations environnementales.

11. Procédé de fonctionnement d'un dispositif électronique (200), le procédé comprenant :
le stockage, dans une mémoire (230), d'un modèle de trouble panique qui est spécifique à l'utilisateur, des informations de trouble panique et d'une pluralité d'informations de guidage pour chacune d'une pluralité de stades de trouble panique ;
la détection d'un état d'un utilisateur par l'intermédiaire d'un module de capteur (276) ;
la détection des informations environnementales par l'intermédiaire d'un module de communication (290) ;
l'entrée, par un processeur (220), de l'état détecté et des informations environnementales détectées dans le modèle de trouble panique pour déterminer un stade de trouble panique d'une pluralité de stades de trouble panique de l'utilisateur, ladite pluralité de stades de trouble panique comprenant un premier stade de trouble panique correspondant à une situation dans laquelle l'utilisateur est exposé à un environnement pouvant entraîner le trouble panique, un deuxième stade de trouble panique correspondant à une situation dans laquelle l'utilisateur est censé générer le trouble panique, un troisième stade de trouble panique correspondant à une situation dans laquelle l'utilisateur présente le trouble panique, et un quatrième stade de trouble panique correspondant à une situation après que le trouble panique survient chez l'utilisateur, la récupération et l'émission en sortie, par l'intermédiaire du montage de circuits de sortie, de l'une de la pluralité d'informations de guidage correspondant au stade de trouble panique déterminé, et
la mise à jour, par le processeur, du modèle de trouble panique sur la base de l'état détecté et des informations environnementales détectées.

12. Procédé de la revendication 11, ledit module de capteur (276) étant configuré pour mesurer un signal lié au mouvement de l'utilisateur ou aux informations biométriques de l'utilisateur, et lesdites informations biométriques comprenant au moins l'un d'un pouls, d'un indicateur de stress, d'un taux de respiration, d'une pression artérielle, d'une température corporelle et d'un taux de sueur.

13. Procédé de la revendication 11, lesdites informations de trouble panique comprenant des emplacements historiques dans lesquels des attaques de panique sont survenues précédemment, lesdites informations environnementales détectées comprenant un emplacement actuel du dispositif électronique (200), et
ledit procédé comprenant en outre :
la détermination du stade de trouble panique par l'intermédiaire du modèle de trouble panique en détectant que l'emplacement actuel correspond à un emplacement historique compris dans les informations de trouble panique.

14. Procédé de la revendication 11, comprenant en outre :
la détection des informations environnementales en balayant, par l'intermédiaire du module de communication (290), un environnement immédiat du dispositif électronique, lesdites informations environnementales comprenant l'identification d'un dispositif électronique externe, et
la détermination du stade de trouble panique sur la base de l'identification détectée du dispositif électronique externe.

15. Procédé de la revendication 14, lesdites informations de trouble panique comprenant une liste d'identifications de dispositif, et
le procédé comprenant en outre :
la détermination du stade de trouble panique sur la base en outre de la détermination pour savoir si l'identification détectée du dispositif électronique externe est comprise dans la liste d'identifications de dispositif.
